# FASCICULE DE BREVET EUROPEEN

(11) **EP 3 655 109 B1**
(45) Date de publication et mention de la délivrance du brevet: **21.04.2021**
(21) Numéro de dépôt: 18750250.5
(22) Date de dépôt: 11.07.2018
(51) Int. Cl.: A61Q 7/00, A61K 8/99, A61P 17/14

(54) **COMPOSITION COSMÉTIQUE OU PHARMACEUTIQUE FAVORISANT LA POUSSE DES CHEVEUX COMPRENANT UN LYOPHILISAT DE CELLULES DÉDIFFÉRENCIÉES DE CRISTE MARINE**
KOSMETISCHE ODER PHARMAZEUTISCHE ZUSAMMENSETZUNG ZUR FÖRDERUNG DES HAARWACHSTUMS MIT EINEM LYOPHILISAT AUS ENTDIFFERENZIERTEN MEERFENCHELZELLEN
COSMETIC OR PHARMACEUTICAL COMPOSITION FOR PROMOTING HAIR GROWTH, COMPRISING A LYOPHILISATE OF DEDIFFERENTIATED SEA FENNEL CELLS

(30) Priorité: 20.07.2017 FR 1756876
(43) Date de publication de la demande: 27.05.2020
(73) Titulaire: BiotechMarine, 22260 Quemper Guezennec (FR); Société d'Exploitation de Produits pour les Industries Chimiques SEPPIC, 75321 Paris cedex 07 (FR)
(72) Inventeur: CATTUZZATO, Laetitia, 81100 Castres (FR); FOUILLAND, Julien, 22260 Quemper Guezennec (FR)
(74) Mandataire: Air Liquide
(86) Numéro de dépôt international: PCT/FR2018/051744
(87) Numéro de publication internationale: WO 2019/016446

(56) Documents cités:
- WO-A2-2010/067212
- WO-A2-2013/120620
- FR-A1- 2 864 446
- FR-A1- 2 985 423
- DATABASE WPI Week 201212 Thomson Scientific, London, GB; AN 2012-B54928 XP002776161, -& JP 2012 020946 A (KRACIE HOME PROD KK) 2 février 2012 (2012-02-02)

## Description

La présente invention a pour objet un nouveau procédé pour favoriser la croissance des cheveux, une nouvelle composition synergique, ainsi que sa mise en œuvre dans ledit procédé.

Les cheveux sont agressés par divers facteurs externes au corps humain qu'il s'agisse de stress mécaniques ou chimiques et notamment de stress dus à la pollution atmosphérique ou aux rayonnements ultra-violets qu'ils soient naturels ou artificiels.

Il est connu que l'homme dispose d'un capital moyen de cinq millions de follicules pileux, un million sur la tête et quatre millions sur le corps. Le cheveu se développe dans l'annexe de la peau appelée follicule pilo-sébacé. Cette annexe comprend le cheveu ou poil en lui-même qui pousse au sein du follicule pileux, auquel est annexée une glande sébacée. Le muscle horripilateur est situé à la base de chaque poil. Il s'étend de la base de la gaine épidermique du poil à l'épiderme de surface de la peau et participe à la thermorégulation au cours du "frisson ou "chair de poule". Chaque follicule est innervé et richement vascularisé, permettant de recevoir la sensation tactile ainsi que d'assurer la croissance des cheveux/poils.

Le follicule pileux se compose principalement d'un bulbe comprenant la papille dermique, les fibroblastes constituant sa population cellulaire, la matrice comprenant des kératinocytes et des mélanocytes et une tige pilaire comprenant trois gaines, épithéliale interne, épithéliale externe et épithéliale conjonctive. Le cheveu pousse selon un cycle de croissance composé de trois phases distinctes, les phases catagène, télogène et anagène. La phase catagène (dite de « mort » du cheveu) est une phase d'une durée de deux à trois semaine, d'involution du follicule pileux au cours de laquelle la majorité des cellules épithéliales du bulbe et de la gaine épithéliale cessent de proliférer et enclenchent une apoptose par laquelle les deux-tiers inférieurs du follicule pileux est affecté. La différenciation cellulaire diminue très fortement jusqu'à s'interrompre. La papille dermique reste en contact avec l'épithélium. Le volume du bulbe décroît et monte vers la surface de l'épiderme jusqu'à se séparer de la papille dermique.

La phase télogène d'une durée de six à sept mois est une phase de latence au cours de laquelle il n'y a nib de différentiation ni de prolifération cellulaire significatives et l'apoptose a cessé. La papille dermique quitte les profondeurs du derme et revient peu à peu en contact avec le reste du bulbe.

La phase anagène d'une durée de deux à quatre ans pour les hommes et jusqu'à six ans pour les femmes est initiée par l'activation des cellules souches épithéliales et progénitrices localisées dans des compartiments cellulaires spécialisés situés dans le bulbe et germe secondaire. Cette activation est suivie d'une une rapide prolifération et différentiation des kératinocytes proches de la papille dermique et conduit à la formation d'un nouveau bulbe de cheveux et à la croissance de la gaine interne.

Ce cycle de croissance se répète entre vingt-cinq et trente fois au cours de la vie, grâce à la capacité régénératrice des cellules souches du follicule qui résident dans la région du bulbe.

Dans le cadre de leurs recherches visant à développer de nouveaux actifs cosmétiques favorisant la pousse des cheveux, les inventeurs se sont intéressés aux cellules végétales halophiles.

C'est pourquoi, selon un premier aspect, l'invention a pour objet un lyophilisat de cellules dédifférenciées de *Crithmum maritimum* (Criste marine), pour son utilisation dans une méthode de traitement thérapeutique destinée à favoriser la pousse des cheveux.

Ledit lyophilisat de cellules dédifférenciées de *Crithmum maritimum,* mis en œuvre dans l'utilisation définie ci-dessus est préparé par des méthodes connues de l'homme du métier et plus particulièrement selon le procédé décrit dans la demande de brevet français publiée sous le numéro 2 864 446, aux pages 7 à 9.

Selon un autre aspect de la présente invention, celle-ci a pour object un procédé non-thérapeutique permettant de favoriser le pousse des cheveux comprenant l'application sur le cuir chevelu d'un composition topique (C₁) comprenant une quantité efficace dudit lyophilisat de cellules dédifférenciées de *Crithmum maritimum* (Criste marine).

Par quantité efficace, on désigne une quantité suffisante qui permette au consommateur d'attention moyenne, de constater visuellement la réalité de l'amélioration invoquée quant à la pousse des ses propres cheveux. L'étude expérimentale exposée dans la suite la présente desciption fait ressotir qu'une quantité comprise entre 0,05% et 0,5% massique dudit lyphilisat, par rapport à la masse totale de ladite composition (C₁), peut être considérée comme une quantité efficace.

Ladite composition topique (C₁) comprenant ledit lyphilisât objet de la présente invention est de préférence sous une forme adaptée au traitement du cuir chevelu et/ou des cheveux, tel que une huile, une crême, un lait, une poudre, une lotion, une formule moussante ou un shampoing. Il peut donc s'agir d'une formulation rincée ou non rincée.

La composition (C₁) objet de la présente invention, se présente donc généralement sous forme d'une solution aqueuse ou hydro-alcoolique ou hydro-glycolique, sous forme d'une suspension, d'une émulsion, d'une microémulsion ou d'une nano-émulsion, qu'elles soient de type eau-dans-huile, huile-dans-eau, eau-dans-huile-dans-eau ou huile-dans-eau-dans-huile, ou sous forme d'une poudre.

La composition (C₁) comprend en outre un ou plusieurs des excipients pharmaceutiquement ou cosmétiquement acceptables.

L'expression « cosmétiquement acceptable » signifie selon la directive du Conseil de la Communauté Economique Européenne N°76/768/CEE du 27 juillet 1976 modifiée par la directive N°93/35/CEE du 14 juin 1993, qu'elle comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

La composition (C₁) objet de la présente invention, peut être conditionnée dans un flacon, dans un dispositif de type "flacon" pompe ou sous forme pressurisées dans un dispositif aérosol.

Comme excipients pharmaceutiquement ou cosmétiquement acceptable, optionnellement présents dans la composition (C₁) telle que définie précédemment il y par exemple les tensioactifs moussants et/ou détergents, les tensioactifs épaississants et/ou gélifiants, les agents épaississants et/ou gélifiants, les agents stabilisants, les composés filmogènes, les solvants et co-solvants, les agents hydrotropes, les eaux thermales ou minérales les agents plastifiants, les agents émulsionnants et co-émulsionnants, les agents opacificants, les agents nacrants, les agents surgraissants, les séquestrants, les agents chélatants, les huiles, les cires, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents que l'on peut associer au lyophilisat de cellules dédifférenciées de Criste marine dans la composition (C₁), on peut citer les tensioactifs moussants et/ou détergents anioniques, cationiques, amphotères ou non ioniques.

Parmi les tensioactifs anioniques moussants et/ou détergents, on peut citer les sels de métaux alcalins, de métaux alcalino-terreux, d'ammonium, d'amines, ou d'aminoalcools d'alkylether sulfates, d'alkyl sulfates, d'alkylamidoéther sulfates, d'alkylaryl polyéthersulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkyl sulfosuccinates, d'alkyléther sulfosuccinates, d'alkylamide sulfosuccinates, d'alkyl sulfoacétates, d'alkyl sarcosinates, d'acyl iséthionates, de N-acyl taurates, d'acyl lactylates, de dérivés N-acylés d'acides aminés, de dérivés N-acylés de peptides, de dérivés N-acylés de protéines, de dérivés N-acylés d'acides gras.

Parmi les tensioactifs amphotères moussants et/ou détergents, on peut citer les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates.

Parmi les tensioactifs cationiques moussants et/ou détergents, on peut citer particulièrement les dérivés d'ammoniums quaternaires.

Parmi les tensioactifs non ioniques moussants et/ou détergents, on peut citer plus particulièrement les alkylpolyglycosides comportant un radical aliphatique, linéaire ou ramifié, saturé ou insaturé, et comportant de 8 à 16 atomes de carbone, comme l'octyl polyglucoside, le décyl polyglucoside, l'undécylényl polyglucoside, le dodécyl polyglucoside, le tétradécyl polyglucoside, l'hexadécyl polyglucoside, le 1-12 dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Comme exemples de tensioactifs épaississants et/ou gélifiants que l'on peut associer au lyophilisat de cellules dédifférenciées de Criste marine dans la composition (C₁), on peut citer les esters gras d'alkylpolyglycosides éventuellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ; les esters gras alcoxylés tels que le PEG 150 pentaérythrytyl tétrastéarate commercialisé sous l'appellation CROTHIX™ DS53, le PEG 55 propylène glycol oléate commercialisé sous l'appellation ANTIL™ 141 ; les carbamates de polyalkylène glycols à chaînes grasses comme le PPG-14 laureth isophoryl dicarbamate commercialisé sous l'appellation ELFACOS™ T211, le PPG-14 palmeth-60 hexyl dicarbamate commercialisé sous l'appellation ELFACOS™ GT2125.

Comme exemples d'agents épaississants et/ou gélifiants que l'on peut associer au lyophilisat de cellules dédifférenciées de Criste marine dans la composition (C₁), on peut citer les polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide 2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- methyl)acrylamido methane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymère linéaire, branché ou réticulé d'au moins un monomère possédant une fonction acide fort, libre, partiellement salifiée ou totalement salifiée, avec au moins un monomère neutre, et au moins un monomère de formule (VIII) :

CH₂=C(R'₃)-C(=O)-[CH₂-CH₂-O]ₙ-R'₄ (VIII)

dans laquelle R'₃ représente un atome d'hydrogène ou un radical méthyle, R'₄ représente un radical alkyle linéaire ou ramifié comportant de huit à trente atomes de carbone et n représente un nombre supérieur ou égal à un et inférieur ou égal à cinquante.

Les polymères de type polyélectrolytes, linéaires ou branchés ou réticulés que l'on peut associer au lyophilisat de cellules dédifférenciées de Criste marine dans la composition (C₁), peuvent se présenter sous la forme d'une solution, d'une suspension aqueuse, d'une émulsion eau-dans-huile, d'une émulsion huile-dans-eau ou d'une poudre de polymère. On peut citer par exemple les produits commercialisés sous les appellations SIMULGEL™ EG, SIMUL-GEL™EPG, SEPIGEL™ 305, SIMULGEL™ 600, SIMULGEL™ NS, SIMULGEL™ INS 100, SIMULGEL™ FL, SIMULGEL™ A, SIMULGEL™ SMS 88, SEPINOV™EMT 10, SE-PIPLUS™400, SEPIPLUS™265, SEPIPLUS™S, SEPIMAX™Zen, ARISTOFLEX™AVC, ARISTOFLEX™AVS, NOVEMER™EC-1, NOVEMER™EC 2, ARISTOFLEX™HMB, COSME-DIA™SP, FLOCARE™ET 25, FLOCARE™ET 75, FLOCARE™ET 26, FLOCARE™ET 30, FLOCARE™ET 58, FLOCARE™PSD 30, VISCOLAM™AT 64, VISCOLAM™AT 100.

Comme exemples d'agents épaississants et/ou gélifiants que l'on peut associer au lyophilisat de cellules dédifférenciées de Criste marine dans la composition (C₁), on peut citer les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1).

Comme exemples d'agents épaississants et/ou gélifiants que l'on peut associer au lyophilisat de cellules dédifférenciées de Criste marine dans la composition (C₁), on peut citer les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes ; la cellulose et les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants que l'on peut associer au lyophilisat de cellules dédifférenciées de Criste marine dans la composition (C₁), on peut citer les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples de solvants que l'on peut associer au lyophilisat de cellules dédifférenciées de Criste marine dans la composition (C₁), on peut citer l'eau, les solvants organiques comme le glycérol, le diglycérol, les oligomères du glycérol, l'éthylène glycol, le propylène glycol, le butylène glycol, le 1,3-propanediol, le 1,2-propanediol, l'hexylène glycol, le diéthylène glycol, le xylitol, l'érythritol, le sorbitol, les alcools hydrosolubles tels que l'éthanol, l'isopropanol ou le butanol, les mélanges d'eau et desdits solvants organiques.

Comme exemples d'eaux thermales ou minérales que l'on peut associer au lyophilisat de cellules dédifférenciées de Criste marine dans la composition (C₁), on peut citer les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Né-ris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fu-mades et l'eau de Tercis-les-bains.

Comme exemples d'agents hydrotropes que l'on peut associer au lyophilisat de cellules dédifférenciées de Criste marine dans la composition (C₁), on peut citer les xylènes sulfonates, les cumènes sulfonates, l'hexyl polyglucoside, le (2-éthyl hexyl) polyglucoside ou le n-heptyl polyglucoside.

Comme exemples d'agents tensioactifs émulsionnants que l'on peut associer au lyophilisat de cellules dédifférenciées de Criste marine dans la composition (C₁), on peut citer les tensioactifs non ioniques, des tensioactifs anioniques, des tensioactifs cationiques.

Comme exemples de tensioactifs non-ioniques émulsionnants que l'on peut associer au lyophilisat de cellules dédifférenciées de Criste marine dans la composition (C₁), on peut citer les esters d'acides gras et de sorbitol, comme les produits commercialisés sous les appellations MONTANE™40, MONTANE™60, MONTANE™70, MONTANE™80 et MON-TANE™85 ; les compositions comprenant du stéarate de glycérol et l'acide stéarique éthoxylé entre cinq moles et cent cinquante moles d'oxyde d'éthylène, comme la composition comprenant de l'acide stéarique éthoxylé à cent trente-cinq moles d'oxyde d'éthylène et du stéarate de glycérol commercialisée sous l'appellation SIMULSOL™ 165 ; les esters de mannitan ; les esters de mannitan éthoxylés ; les esters de sucrose ; les esters de méthyl glucoside ; les alkyl polyglycosides comportant un radical aliphatique, linéaire ou ramifié, saturé ou insaturé, et comportant de quatorze à trente-six atomes de carbone, comme le tétradécyl polyglucoside, l'hexadécyl polyglucoside, l'octadécyl polyglucoside, l'hexadécyl polyxyloside, l'octadécyl polyxyloside, l'eicosyl polyglucoside, le dodécosyl polyglucoside, le (2-octyl dodécyl) polyxyloside, le (12-hydroxy stéaryl) polyglucoside ; les compositions d'alcools gras linéaires ou ramifiés, saturés ou insaturés, et comportant de quatorze à trente-six atomes de carbone, et d'alkyl polyglycosides tels que décrits précédemment, par exemple les compositions commercialisées sous les noms de marque MONTANOV™68, MONTANOV™14, MONTANOV™82, MONTA-NOV™202, MONTANOV™S, MONTANOV™WO18, MONTANOV™L, FLUIDANOV™20X et EASYNOV™.

Comme exemples de tensioactifs anioniques que l'on peut associer au lyophilisat de cellules dédifférenciées de Criste marine dans la composition (C₁), on peut citer le glycéryl stéarate citrate, le cétéarylsulfate, les savons comme le stéarate de sodium ou le stéarate de triéthanolammonium, les dérivés N-acylés d'acides aminés salifiés comme par exemple le stéaroyl glutamate.

Comme exemples de tensioactifs cationiques émulsionnants que l'on peut associer au lyophilisat de cellules dédifférenciées de Criste marine dans la composition (C₁), on peut citer les aminoxydes, le quaternium-82 et les tensioactifs décrits dans la demande de brevet WO 96/00719 et principalement ceux dont la chaîne grasse comprend au moins seize atomes de carbone.

Comme exemples d'agents opacifiants et/ou nacrants que l'on peut associer au lyophilisat de cellules dédifférenciées de Criste marine dans la composition (C₁), on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de douze à vingt-deux atomes de carbone.

Comme exemples d'agents de texture que l'on peut associer au lyophilisat de cellules dédifférenciées de Criste marine dans la composition (C₁), on peut citer des dérivés N-acylés d'acides aminés, comme la lauroyl lysine commercialisée sous l'appellation AMINOHOPE™LL, l'octenyl starch succinate commercialisé sous l'appellation DRYFLO™, le myristyl polyglucoside commercialisé sous l'appellation MONTANOV™ 14, les fibres de cellulose, les fibres de coton, les fibres de chitosane, le talc, la séricite, le mica.

Comme exemples d'huiles que l'on peut associer au lyophilisat de cellules dédifférenciées de Criste marine dans la composition (C₁), on peut citer les huiles minérales telles que l'huile de paraffine, l'huile de vaseline, les isoparaffines ou les huiles blanches minérales ; les huiles d'origine animale, telles que le squalène ou le squalane ;les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de car-thame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires que l'on peut associer au lyophilisat de cellules dédifférenciées de Criste marine dans la composition (C₁), on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de principes actifs que l'on peut associer au lyophilisat de cellules dédifférenciées de Criste marine dans la composition (C₁), on peut citer les composés suivants
- Les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ;
- les acides α-aminés naturels N-acylés pour lesquels le radical acyle comprend de huit à dix-huit atomes de carbone; plus généralement les composés de formule (I):

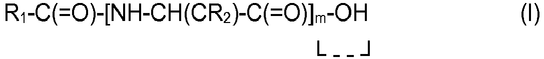

Ou des mélanges de dits composés de formules (I) tels que ceux décrits dans la demande internationale publiée sous le numéro WO 94/26694 ou commercialisés sous les noms PROTEOL™ SAV 505 ou PROTEOL™ OAT.
- Les extraits végétaux riches en polyphénols comme les extraits de raisin, les extraits de pin, les extraits de vin, les extraits d'olives ;
- Les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides
   les agents destinés au traitement des cheveux et/ou des poils, par exemple des agents protecteurs des mélanocytes du follicule pileux, destinés à protéger lesdits mélanocytes contre les agents cytotoxiques responsables de la sénescence et/ou de l'apoptose desdits mélanocytes, tels que les agents mimétiques de l'activité de la DOPAchrome tautomérase choisis parmi ceux décrits dans la demande de brevet européen publiée sous le numéro EP 1 515 688 A2, les molécules synthétiques mimétiques de la SOD par exemples les complexes de manganèse, des composés antioxydants par exemple les dérivés de cyclodextrine, des composés silicés dérivés d'acide ascorbique, de la pyrrolidone carboxylate de lysine ou d'arginine, des associations de mono- et diester d'acide cinnamique et de vitamine C.
- Les agents tensioactifs à groupement phosphate de la famille de polysiloxanes alkoxylé et phosphatés de formule (II) :

   ZO-(CH₂)₃-NH-C(=O)-CH(CH₃)₂-CH₂-OH (II)
dans laquelle Z représente un radical dérivé du phosphore de formule : P(R₃O)(OM)(=O)- dans lequel R3 représente un dérivé de polysiloxanes alcoxylés et OM un radical OH libre ou salifié; comme celui commercialisé sous le nom PECOSIL™ SPP 50 (dénomination INCI: Potassium dimethicone copolyol pathènyl phosphate);

Comme exemples d'agents antioxydants que l'on peut associer au lyophilisat de cellules dédifférenciées de Criste marine dans la composition (C1), on peut citer l'EDTA et ses sels, l'acide citrique, l'acide tartarique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE™ GL 47S commercialisé par la société Akzo Nobel sous le nom INCI : Tetrasodium Glutamate Diacetate.

Selon un aspect plus particulier de la présente invention, celle-ci a pour objet une composition topique (C'₁) comprenant pour 100% massique :
- De 0,05% massique à 0,5% massique dudit lyophilisat de cellules dédifférenciées de Criste marine ;
- De 0,01% massique à 5% massique d'au moins un les acides α-aminés naturels N-acylés pour lesquels le radical acyle comprend de dix ou douze atomes de carbone;
- De 0.01% massique à 5% d'au moins un agent tensioactif à groupement phosphate de la famille de polysiloxanes alkoxylé et phosphatés de formule (II) :

   ZO-(CH₂)₃-NH-C(=O)-CH(CH₃)₂-CH₂-OH (II)
dans laquelle Z représente un radical dérivé du phosphore de formule : P(R₃O)(OM)(=O)- dans lequel R3 représente un dérivé de polysiloxanes alcoxylés et OM un radical OH libre ou salifié; son utilisation en cosmétique.

L'invention a aussi pour objet la composition C'1) telle que définie ci-dessus, pour son utilisation dans une méthode de traitement thérapeutique destinée à favoriser la pousse des cheveux; ainsi qu'un procédé non-thérapeutique permettant de favoriser le pousse des cheveux comprenant l'application sur le cuir chevelu de la composition topique (C'₁) telle qu définie ci-dessus.

La partie expérimentale suivante illustre l'invention sans toutefois la limiter.

### 1. Choix du modèle et pertinence

Le modèle choisi pour mettre en évidence l'effet technique du lyophilisat objet de la présente invention, est un modèle d'étude de l'expression génique sur des explants de liftings humains. Par « liftings humains », on entend au sens de la présente invention des déchets de peaux humaines provenant de prélèvements cutanés situés vers la tempe et le front, obtenus lors d'opérations de chirurgie esthétique ; ces « déchets de peau humaine » constituent des modèles de choix lorsque l'on veut étudier l'efficacité d'ingrédients sur le système pileux. De plus, les analyses génétiques dites « full genome » permettent de mettre en évidence l'ensemble des régulations induites par un composé.

Par « analyses génétiques dites « full genome » », on entend au sens de la présente invention, les analyses mettant en œuvre des méthodes de biologie moléculaire appliquées à l'ensemble du génome humain pour déterminer en une seule expérimentation la variation d'expression de l'ensemble des gènes et des séquences génétiques du génome humain, tant sur les parties desdits gènes et desdites séquences génétiques codant des protéines et sur les parties non codantes de protéines. De telles analyses génétiques dites « full genome » permettent d'envisager une vision globale de l'efficacité d'une substance chimique ou d'une composition chimique au regard de propriétés physiologiques potentielles et d'appréhender l'ensemble des régulations biologiques du corps humain. Dans le cas présent, de telles analyses génétiques dites « full genome » permettent d'estimer l'existence ou non de propriétés biologiques associées aux lyophilisats de cellules dédifférenciées testés dans des conditions de traitement déterminées (application par voie topique, durée de traitement,..), par comparaison entre les résultats obtenus sur les explants traités avec les lyophilisats de cellules dédifférenciées testés et les explants traités avec la formulation placebo, selon la même méthode expérimentale

### 2. Protocole expérimental

Trois explants de 0,5cm² issus d'un lifting d'un homme caucasien âgé de soixante-deux ans sont mis en culture.

On prépare des gels hydro-alcooliques à 2,5% d'éthanol (Vol./Vol.) contenant ou non (contrôle) le lyophilisat de cellules dédifférenciées de *Crithmum maritimum* (L₁) à 0,1% massique.

On applique les gels à hauteur de 2mg/explant à t = 0.

A t = 24 heures, les trois explants traités sont coupés en 3 parties :
Une première partie est utilisée en extraire les ARN pour l'étude transcriptonique, elle est plongée dans le milieu (RNAlater® RNA Stabilization Reagent), commercialisé par la société Qiagen, qui est un milieu tampon destiner à stabiliser les ARNs, pour éviter leur dégradation ;

Une deuxième partie est congelée à - 80°C pour une étude histologique;

Une troisième partie est mise en solution de formaline également pour une étude histologique.

A t = 2 jours, t = 5 jours et t = 7 jours, les gels sont de nouveau appliqués sur les explants restant et à t = 8 jours les cultures sont stoppées.

Chaque explant est ensuite coupé en 2 puis fixé soit à - 80°C, soit en solution de formaline pour les études histologiques.

L'analyse transcriptomique est effectuée après extraction des "ARNm", vérification de leur qualité, suivie d'une étape de transcription inverse, puis amplifiée avant d'être hybridés sur la puce full genome "Whole Human Genome Microarray (8*60K)", qui contient 62 976 sondes géniques.

Par « analyse transcriptomique », on entend au sens de la présente invention la méthode permettant de caractériser et de quantifier l'ensemble des ARNs issus de la transcription du génome. L'analyse transcriptomique permet de caractériser l'ensemble des ARNs issus de la transcription du génome d'un tissu particulier, d'un type cellulaire particulier ou de comparer les ARNs transcris entre différentes conditions expérimentales. Dans le cas présent, l'analyse transcriptomique est conduite sur les explants de « lifting », tel que défini précédemment, pour identifier la qualitié et l'ampleur de la transcription des ARNs liés à la pousse du cheveu. Cette méthode appliquée dans le cadre de la présente invention a pour but d'évaluer en une seule expérimentation l'ensemble des régulations génétiques humaines impliquées dans la pousse du cheveu pour des explants mis en présence du placébo et mis en présence des lyophilisats de cellules dédifférenciées de *Crithmum maritimum.*

Par « vérification de leur qualité », on entend au sens de la présente demande la démarche qui consiste à s'assurer que les quantités et la qualité des ARNm qui sont obtenus après extraction, permettent à la fois de disposer d'une quantité suffisante pour poursuivre l'expérimentation et de déceler les gènes même faiblement exprimés ; l'aspect qualitatif, mené selon les standards en vigueur et connus de l'homme du métier, permet de confirmer l'absence de dégradation des ARN.

Par étape de « transcription inverse », on entend au sens de la présente invention l'étape connue de l'homme du métier et consistant à transformer les ARN extraits en ADN complémentaires.

Par étape « d'amplification » des ADN complémentaires ainsi obtenus, on entend au sens de la présente invention la technique permettant d'amplifier un exemplaire ou quelques segment d'ADN complémentaire en millions de copies de la séquence génétique concernée, et plus particulièrement la technique dite « PCR » (ou « Polymerase Chain Reaction » ou « réaction en chaine par polymérase »).

Par étape « d'hybridation sur une puce», on entend au sens de la présente invention l'installation des brins d'ADN précédemment amplifiés sur les puces dites "full genome "Whole Human Genome Microarray (8*60K)" sur lesquelles sont greffées des fragments génétiques représentatifs de l'ensemble du génome humain. Lorsque une séquence d'ADN provenant de l'étape d'amplification se trouve en présence d'une même séquence d'ADN présente sur la puce, ces séquences s'apparient (hybridation). La quantité d'hybridation ou d'appariement est estimée être proportionnelle à la quantité d'ARN présente au départ de l'expérimentation, et par conséquent, le signal de détection des hybridations ou appariements sera proportionnel au_niveau d'expression du gène dans le système biologique de départ (provenant de l'explant traité ou non).

Par « puce full genome "Whole Human Genome Microarray (8*60K)", on entend au sens de la présente invention un support, généralement en verre, sur lequel sont fixés les fragments d'ADN représentatifs de l'ensemble du génome humain, commercialisé par la société Agilent.

Après la validation qualitative des puces telles que précédemment décrites, et la normalisation des données est réalisée selon des méthodes connues de l'homme du métier (normalisation par rapport aux gènes dits de « ménage » dont l'expression ne varie pas dans les mêmes conditions expérimentales), de façon à déterminer pour chaque gène un facteur de variation, nommé « fold change (FC) », caractéristique du ratio « intensité du niveau d'expression du gène traité par la présence des lyophilisats de cellules dédifférenciées de *Crithmum maritimum* » sur « intensité du niveau d'expression du gène traité par la formulation placébo ».

Dans le cadre de l'interprétation des résultats provenant de la mise en œuvre de cette méthode d'évaluation expérimentale, on estimera que lorsque le facteur de variation (ou « Fold Change (FC) ») tel que défini ci-dessus, est supérieur ou égal à une valeur de 1,45, la surexpression du gène concerné sera jugée comme significative, et lorsque la valeur du facteur de variation (ou « Fold Change (FC) ») tel que défini ci-dessus est inférieure ou égale à 0,65 la répression du gène concerné sera jugée comme significative.

Les connaissances générales de l'homme du métier, ont permis à la demanderesse de dresser le Tableau 1 (ci-dessous) reliant des gènes spécifiques aux protéines qu'ils encodent, et à l'action de ces protéines sur le métabolisme particulier du cycle de vie du follicule pileux.

**Tableau 1**

| **Gène/Protéines encodées par le gène** | **Rôle physiologique de la protéine encodée** | **Lien de la protéine encodée avec le cycle de vie du follicule pileux** |
|---|---|---|
| KRT 19/Kératine 19 | Protéine du cytosquelette des kératinocytes | Marqueur des cellules souches du follicule pileux, et indicateur du renouvellement des cellules souches du follicule pileux |
| ITGA6/Intégrine α-6 | Protéine agissant comme récepteur membranaire aux laminnines (liens entre les cellules et la matrice) | Activation de la voie PI3K/AKT permettant l'induction d'un nouveau cycle du follicule pileux et la prolifération des progéniteurs épidermiques des interfollicules et de la gaine du follicule |
| ITGA3/Intégrine α-3 | Protéine agissant comme récepteur membranaire aux laminnines (liens entre les cellules et la matrice) | Prolonge la phase anagène, induit la prolifération cellulaire, améliore l'intégrité structurale du follicule pileux |
| CA6/Carbonic anhydrase 6 | Enzymes impliquées dans l'équilibre acide-base | L'enzyme CA6 favorise la croissance du follicule pileux lorsque surexprimée en phase anagène |
| CA9Carbonic anhydrase 9 | Enzyme impliquée dans l'équilibre acide-base | L'enzyme CA9 permet la régulation du pH lors de la prolifération et la différentiation cellulaire |
| CYP27B1/Cytochrome P450 family 27B | Enzyme impliquée dans la synthèse de la vitamine D3 | La vitamine D initie le cycle de vie du follicule pileux, via l'activation de la voie Wnt; agit pour la régulation du métabolisme du calcium dans la fonction barrière épidermique, et agit comme marqueur différentiel et dans la synthèse du cholestérol et d'autres lipides |
| NAMPT : Nicotanamide Phosphoribosyltransferase ADAM 15: ADAM metallopeptidase domain 15 | DNER = ligand Notch - NAMPT : facteur d'homéostasie NAD (Nicotinamide dinucléotide) ADAM : facteur d'adhésion cellules/cellules | Implication dans l'induction de la voie Notch qui permet l'induction de la croissance du follicule pileux (différentiation/prolifération des cellules souches) et le maintien de la phase anagène |

### 3. Résultats

Les cellules dédifférenciées de Criste marine, appliquées par voie topique sur des explants de « Lifting », tels que définis précédemment, en une quantité massique de 0,1% par l'intermédiaire d'un gel hydro-alcoolique, ont permis d'induire la surexpression de 44 gènes et la répression de 34 gènes, suite à la mise en œuvre de la méthode d'analyse transcriptomique décrite ci-dessus.

Parmi ces 44 gènes surexprimés et ces 34 gènes réprimés, on a consigné dans le Tableau 2, les valeurs des facteurs de variation (ou « Fold Change (FC) ») entre le niveau d'expression de l'explant traité avec le lyophilisat de cellules dédifférenciées de Criste marine et le niveau d'expression de la formule placébo, pour les gènes pertinents dans les mécanismes biologiques et physiologiques liés à la repousse du cheveu ou au cycle de vie du follicule pileux.

**Tableau 2**

| **Identité du gène** | **Valeur facteur de variation (ou « Fold Change (FC) ») mesurée** |
|---|---|
| KRT 19 | 1,74 |
| ITGA 3 | 2,00 |
| ITGA 6 | 1,87 |
| CYP27B1 | 1,56 |
| CA6 | 2,08 |
| CA9 | 2,68 |
| DNER | 1,95 |
| NAMPT | 1,58 |
| ADAM15 | 1,93 |

On peut donc conclure de ces résultats expérimentaux que l'application du lyophilisat de cellules dédifférenciées de Criste marine permet la protection et la prolifération des cellules souches du follicule pileux :
- de favoriser et de protéger le renouvellement des cellules souches du follicule pileux (surexpression de KRT19),
- de permettre la prolifération et la différenciation cellulaire par la régulation des conditions de pH (surexpression de CA9)
- de permettre la prolifération des progéniteurs épidermiques des interfollicules et de la gaine du follicule (surexpression de ITGA6)

On peut également conclure de ces résultats expérimentaux que l'application du lyophilisat de cellules dédifférenciées de Criste marine permet également :
- de favoriser le maintien de la phase de croissance du cheveu ou phase anagène (surexpression d'ITGA3 et de CA6),
- de favoriser l'initiation de nouveaux follicules pileux (surexpression de ITGA6, de DNER/ADAM15).

La conduite de la méthode d'analyse transcriptomique décrite ci-dessus a également permis d'observer des valeurs de facteurs de variation (ou « Fold Change (FC) ») entre le niveau d'expression de l'explant traité avec le lyophilisat de cellules dédifférenciées de Criste marine et le niveau d'expression de la formule placébo, pour des gènes liés à des activités de protection de l'organisme humain contre des activités oxydantes, contre des agressions microbiennes, et pour des gènes liés à des régulations épigénétiques.

Le Tableau 3 contient les connaissances générales de l'homme du métier reliant des gènes spécifiques aux protéines qu'ils encodent, et à l'action de ces protéines sur le métabolisme lié à des activités anti-oxydantes, des activités anti-microbiennes et des activités de régulation épigénétique.

**Tableau 3**

| **Gène/Protéines encodées par le gène** | **Rôle physiologique de la protéine encodée** | **Lien de la protéine encodée avec une activité spécifique** |
|---|---|---|
| PI3 / Peptidase inhibitor 3/Elafin | Enzyme inhibitrice de protéase de type élastase. | Rôle de peptide antimicrobien contre bactéries Gram+/-, protège les fibres élastiques de leur dégradation suite aux UV. Participe à la fonction barrière de la peau. |
| LNC2 / Lipocalin 2 | Protéine de séquestration du fer folliculaire. | Défense contre les infections bactériennes (interfère avec l'acquisition du fer bactérien). Surexprimé en phase anagène du cycle |
| PIGR / Polymeric immunoglobin receptor | Récepteur aux immunoglobulines de type A et M. | Impliqué dans la protection des muqueuses. Participe aux défenses immunitaires innées et adaptatives |
| XDH / *Xanthine dehydrogenase:* | Impliqué dans le cycle redox dans stress oxydatif. | |
| SOD2/*Superoxyde dismutase 2* | Enzyme anti-oxydante | |
| GPX1 et GPX2 / *Gluthatione peroxidase :* | Enzymes anti-oxydantes | |
| NEAT 1/ *Nuclear paraspeckle assembly transcript 1* | Rôle dans la formation des paraspeckles | Contrôle la transcription de gènes cibles par activité épigénétique en séquestrant les protéines de liaison à l'ARN dans des paraspeckles. |

Le Tableau 4 contient les valeurs des facteurs de variation (ou « Fold Change (FC) ») entre le niveau d'expression de l'explant traité avec le lyophilisat de cellules dédifférenciées de Criste marine et le niveau d'expression de la formule placébo, pour des gènes relatifs à des activités anti-microbiennes, des activités anti-oxydantes et des activités de régulation épigénétiques, précisés dans le tableau 3.

**Tableau 4**

| **Identité du gène** | **Valeur facteur de variation (ou « Fold Change (FC) ») mesurée** |
|---|---|
| PI3 | 4,29 |
| LCN2 | 2,51 |
| PIGR | 2,06 |
| XDH | 2,36 |
| SOD2 | 1,57 |
| GPX1 | 1,56 |
| GPX2 | 1,70 |
| NEAT1 | 2,2 |

On peut donc conclure de ces résultats expérimentaux que l'application du lyophilisat de cellules dédifférenciées de Criste marine permet :
- d'apporter une protection globale anti-oxydante (surexpression significative de XDH et GPX2) et plus particulièrement de limiter les mécanismes d'oxydation régissant la canitie,
- de développer une protection face aux agressions bactériennes qui fragilisent la barrière cutanée notamment en phase anagène (surexpression des gènes PI3, LCN2 et PIGR),
- de contrôler l'expression de gènes au cours de la phase de différenciation cellulaire et la réponse aux stress (surexpression NEAT1).

En conclusion, l'application du lyophilisat de cellules dédifférenciées de Criste marine permet de favoriser directement la pousse des cheveux, et la protection descheveux et du cuir chevelu des stresses oxydants et des agressions bactériennes.

Une nouvelle application des cellules dédifférenciées de *Crithmum maritimum* a été mise en évidence. Ainsi, en plus des propriétés dépigmentantes et cicatrisantes déjà décrites, ces cellules présentent également une activité sur la croissance des cheveux, à une dose de 0,1%.

### Exemple 1 : Lotion capillaire thermoactive

### Formule

| | |
|---|---|
| Butylène glycol : | 3,0% |
| SIMULSOL ™1293 : | 3,0% |
| Lyophilisat (L₁) : | 1,0% |
| Acide lactique : | QS pH = 6 |
| SEPICIDE™ HB: | 0,2% |
| SEPICIDE™CI : | 0,3% |
| Parfum : | 0,3% |
| Eau : | QSP 100% |

### Exemple 3 : Shampooing protecteur et relaxant

### Formule

| | |
|---|---|
| Amonyl™ 675 SB : | 5,0% |
| Sodium lauryl éther sulfate à 28% : | 35,0% |
| Lyophilisat (L₁) : | 1,0% |
| Capigel™ 98 : | 3,0% |
| SEPICIDE™ HB: | 0,5% |
| SEPICIDE™CI : | 0,3% |
| Soude : | QS pH = 7,2 |
| Parfum : | 0,3% |
| Colorant (FDC bleu 1/jaune 5) : | QS |
| Eau : | QSP 100% |

### Caractéristiques

Le shampooing obtenu a un aspect limpide vert. Son pH est environ égal à 7.2 et sa viscosité est égale à 1000 cps (BROOKFIELD™ LVT : M4 V6).

### Exemple 4 : Protecteur "leave-on" ; Soin anti-stress pour cheveux

### Formule

| | |
|---|---|
| KETROL ™T: | 0,5% |
| Lyophilisat (L₁) : | 3,0% |
| Butylèneglycol : | 5,0% |
| DC 1501 : | 5,0% |

| | |
|---|---|
| SlMULGEL™EG : | 4,0% |
| SEPICIDE™ HB: | 0,5% |
| SEPICIDE™CI : | 0,3% |
| Parfum : | 0,3% |
| Eau : | QSP 100% |

### Caractéristiques

Le soin obtenu est sous forme de gel opaque. Son pH est environ égal à 6,5 et sa viscosité est égale à 40 000 cps (BROOKFIELD™ LVT : M4 V6).

### Exemple 5 : Masque crème "rince off" restructurant pour cheveux stressés et

### fragilisés

### Formule

| | |
|---|---|
| KETROL™T : | 0,5% |
| Lyophilisat (L₁) : | 3,0% |
| Butylèneglycol : | 3,0% |
| SlMULGEL™EG : | 1,0% |
| MONTANOV™82 : | 3,0% |
| Huile de jojoba : | 1,0% |
| LANOL™P : | 6,0% |
| AMONYL™DM : | 1,0% |
| LANOL™99 : | 5,0% |
| SlMULGEL™EG : | 4,0% |
| SEPICIDE™ HB: | 0,3% |
| SEPICIDE™CI : | 0,2% |
| Parfum : | 0,2% |
| Eau : | QSP 100% |

### Caractéristiques

Le masque obtenu est sous forme de crème. Son pH est environ égal à 6,2 et sa viscosité est égale à 40 000 cps (BROOKFIELD™ LVT : M4 V6).

Les définitions des produits commerciaux utilisés dans les exemples sont les suivantes
SIMULSOL™ 1293 est de l'huile de castor hydrogénée et éthoxylée, avec un indice d'éthoxylation égal à 40, commercialisé par la société SEPPIC.
SEPICIDE™ HB est un mélange conservateur comprenant du Phenoxyethanol, du méthylparaben, de l'éthylparaben, du propylparaben et du butyl paraben, commercialisé par la société SEPPIC.
SEPICIDE™ CI est de l'imidazolidinyl urée, commercialisé par la société SEPPIC. CAPIGEL™ 98 est un épaississant liquide à base de copolymère acrylate commercialisé pas la société SEPPIC.
AMONYL™ 675SB est une sulfobétaïne commercialisée par la société SEPPIC. SIMULGEL™EG est un latex inverse de copolymère (dénomination INCI : Sodium acrylate/Sodium acryloyldimethyltaurate copolymer and Isohexadecane and Polysorbate 80) commercialisé par la société SEPPIC.
KETROL™T est de la gomme de xanthane commercialisée par la société KELCO. LANOL™99 est de l'isononyl isononanoate commercialisé par la société SEPPIC.
DC1501 est un mélange de cyclopentasiloxane et de diméthiconol commercialisé par la société DOW CHEMICAL.
MONTANOV™82 est un agent émulsionnant à base d'alcool cétéarylique et de cocoylglucoside.

## Revendications

1. Lyophilisat de cellules dédifférenciées de *Crithmum maritimum,* pour son utilisation dans une méthode de traitement thérapeutique destinée à favoriser la pousse des cheveux.

2. Procédé non-thérapeutique permettant de favoriser la pousse des cheveux comprenant l'application sur le cuir chevelu d'un composition topique (C₁) comprenant une quantité efficace du lyophilisat de cellules dédifférenciées de *Crithmum maritimum.*

3. Composition topique (C'₁) comprenant pour 100% massique :
- De 0,05% massique à 0,5% massique du lyophilisat de cellules dédifférenciées de *Crithmum maritimum ;*
- De 0,01% massique à 5% massique d'au moins un les acides α-aminés naturels N-acylés pour lesquels le radical acyle comprend de dix ou douze atomes de carbone;
- De 0.01% massique à 5% d'au moins un agent tensioactif à groupement phosphate de la famille de polysiloxanes alkoxylé et phosphatés de formule (II) :
ZO-(CH₂)₃-NH-C(=O)-CH(CH₃)₂-CH₂-OH (II)
dans laquelle Z représente un radical dérivé du phosphore de formule : P(R₃O)(OM)(=O)- dans lequel R₃ représente un dérivé de polysiloxanes alcoxylés et OM un radical OH libre ou salifié;

4. Utilisation non-thérapeutique de la composition (C'1) telle que définie à la revendication 3, en cosmétique.

5. Composition (C'1) telle que définie à la revendication 3, pour son utilisation dans une méthode de traitement thérapeutique destinée à favoriser la pousse des cheveux.

6. Procédé non-thérapeutique permettant de favoriser la pousse des cheveux comprenant l'application sur le cuir chevelu de la composition topique (C'1) telle que définie à la revendication 3.

## Patentansprüche

1. Lyophilisat von undifferenzierten *Crithmum-maritimum-*Zellen zur Verwendung bei einem Verfahren zur therapeutischen Behandlung zur Förderung des Wachstums von Haaren.

2. Nichttherapeutisches Verfahren zur Förderung des Wachstums von Haaren, umfassend das Aufbringen einer topischen Zusammensetzung (C₁), die eine wirksame Menge des Lyophilisats von undifferenzierten *Crithmum-maritimum-*Zellen umfasst, auf die Kopfhaut.

3. Topische Zusammensetzung (C'₁), umfassend pro 100 Massen-%:
- 0,05 Massen-% bis 0,5 Massen-% Lyophilisat von undifferenzierten *Crithmum-maritimum-*Zellen;
- 0,01 Massen-% bis 5 Massen-% mindestens einer der N-acylierten natürlichen α-Aminosäuren mit zehn oder zwölf Kohlenstoffatomen im Acylrest;
- 0,01 Massen-% bis 5 % mindestens eines Tensids mit einer Phosphatgruppe aus der Familie der alkoxylierten und phosphatierten Polysiloxane der Formel (II):
ZO-(CH₂)₃-NH-C(=O)-CH(CH₃)₂-CH₂OH (II)
,
wobei Z für einen von Phosphor abgeleiteten Rest der Formel P (R₃O) (OM) (=O)- steht, wobei R₃ für ein Derivat von alkoxylierten Polysiloxanen steht und OM für einen freien oder versalzten OH-Rest steht.

4. Nichttherapeutische Verwendung der Zusammensetzung (C'1) gemäß Anspruch 3 in der Kosmetik.

5. Zusammensetzung (C'1) gemäß Anspruch 3 zur Verwendung bei einem Verfahren zur therapeutischen Behandlung zur Förderung des Wachstums von Haaren.

6. Nichttherapeutisches Verfahren zur Förderung des Wachstums von Haaren, umfassend das Aufbringen einer topischen Zusammensetzung (C'₁) gemäß Anspruch 3 auf die Kopfhaut.

## Claims

1. Lyophilizate of undifferentiated *Crithmum maritimum* cells, for its use in a therapeutic treatment method for promoting hair growth.

2. Non-therapeutic process for promoting hair growth, comprising the application to the scalp of a topical composition (C₁) comprising an effective amount of the lyophilizate of undifferentiated *Crithmum maritimum* cells.

3. Topical composition (C'₁) comprising, per 100% by mass:
- from 0.05% by mass to 0.5% by mass of the lyophilizate of undifferentiated *Crithmum maritimum* cells;
- from 0.01% by mass to 5% by mass of at least one of the N-acyl natural α-amino acids for which the acyl radical comprises from 10 to 12 carbon atoms;
- from 0.01% by mass to 5% of at least one surfactant bearing a phosphate group of the alkoxylated and phosphated polysiloxane family of formula (II):
ZO-(CH₂)₃-NH-C(=O)-CH(CH₃)₂-CH₂-OH (II)
in which Z represents a phosphorus-based radical of formula: P(R₃O)(OM)(=O)- in which R₃ represents an alkoxylated polysiloxane derivative and OM represents a free or salified OH radical.

4. Non-therapeutic use of the composition (C'1) as defined in Claim 3, in cosmetics.

5. Composition (C'1) as defined in Claim 3, for its use in a therapeutic treatment method for promoting hair growth.

6. Non-therapeutic process for promoting hair growth, comprising the application to the scalp of the topical composition (C'₁) as defined in Claim 3.
